# EUROPEAN PATENT SPECIFICATION

(11) **EP 2 916 883 B1**
(45) Date of publication and mention of the grant of the patent: **01.04.2020**
(21) Application number: 13792184.7
(22) Date of filing: 04.11.2013
(51) Int. Cl.: A61L 31/04

(54) **METHOD OF MANUFACTURING A STENT-GRAFT PROSTHESIS WITH TWO LAYERS OF EXPANDED POLYTETRAFLUOROETHYLENE**
VERFAHREN ZUR HERSTELLUNG EINER STENTGRAFT-PROTHESE MIT ZWEI LAGEN VON EXPANDIERTEM POLYTETRAFLUORETHYLEN
PROCÉDÉ DE FABRICATION D'UNE PROTHÈSE STENT-GREFFON AVEC DEUX COUCHES DE POLYTÉTRAFLUOROÉTHYLÈNE EXPANSÉ

(30) Priority: 12.11.2012 US 201213674404
(43) Date of publication of application: 16.09.2015
(73) Proprietor: Medtronic Vascular Inc., Santa Rosa, CA 95403 (US)
(72) Inventor: BANKS, Andrew, Santa Rosa, CA 95403 (US); MALIK, Asim, Santa Rosa, CA 95403 (US); PEARSON, Meghan, Santa Rosa, CA 95403 (US); DAVIDIAN, Christyne, Santa Rosa, CA 95403 (US)
(74) Representative: Zimmermann & Partner Patentanwälte mbB
(86) International application number: PCT/US2013/068346
(87) International publication number: WO 2014/074464

(56) References cited:
- WO-A1-01/26582
- WO-A1-98/38947
- WO-A2-98/26731
- WO-A2-03/057075
- US-A1- 2001 039 446
- US-B2- 6 547 814

## Description

### FIELD OF THE INVENTION

The invention, as defined by the claims, relates generally to methods of manufacturing a stent-graft prosthesis formed with two layers of expanded polytetrafluoroethylene (ePTFE).
US 2001/039446 A1 describes an encapsulated intraluminal stent-graft and methods of making same. WO 98/38947 A1 describes a conformal laminate stent device. WO 03/057075 A2 describes a hybrid intravascular stent. US 6 547 814 B2 describes selective adherence of stent-graft coverings.

### BACKGROUND OF THE INVENTION

Aneurysms result from weak blood vessel walls which can balloon due to aging and disease and pressure in the vessel. In addition, aneurysmal vessels have a potential to rupture, causing internal bleeding and potentially life threatening conditions. Grafts are used to isolate aneurysms or other blood vessel abnormalities from normal blood pressure, reducing pressure on the weakened vessel wall and reducing the chance of vessel rupture. A tubular endovascular graft is placed within the aneurysmal blood vessel to create a new flow path and an artificial flow conduit through the aneurysm, thereby reducing if not nearly eliminating the exertion of blood pressure on the aneurysm. The graft typically incorporates or is combined with one or more radially expandable stent(s) to be radially expanded in situ to anchor the tubular graft to the wall of the blood vessel at sites upstream and downstream of the aneurysm. Thus, endovascular grafts are typically held in place by mechanical engagement and friction because of the force of the self-expanding or balloon expandable stents.

Expanded polytetrafluoroethylene or ePTFE is a polymeric material that may be used as the graft material of a stent-graft prosthesis. It is known in the art to form a stent-graft prosthesis that includes a sheet or film of ePTFE which covers or lines at least one stent, as described in, for example, U.S. Pat. Nos. 5,700,285 and 5,735,892 to Myers et al. It is also known in the art to form a stent-graft prosthesis that includes an inner PTFE tubular structure, an outer PTFE tubular structure positioned about the inner PTFE tubular structure and at least one stent interposed or encapsulated between the inner and outer PTFE tubular structures, as described in, for example, U.S. Pat. No. 6,673,103 to Golds et al.

Embodiments hereof relate to methods of manufacture for a stent-graft prosthesis having at least one stent positioned between or encapsulated within two layers of ePTFE, wherein the methods utilize direct heating elements to reduce baking temperatures and/or reduce baking times required to couple the two layers of ePTFE together. In addition, the methods described herein may be utilized to form a stent-graft prosthesis without an intermediary adhesive or melt layer between the two layers of ePTFE.

Brief Summary of the Invention The invention relates to a method of manufacturing a stent-graft prosthesis in accordance with appended claims 1 and 7.

### BRIEF DESCRIPTION OF DRAWINGS

The foregoing and other features and advantages of the invention will be apparent from the following description of embodiments hereof as illustrated in the accompanying drawings. The accompanying drawings, which are incorporated herein and form a part of the specification, further serve to explain the principles of the invention and to enable a person skilled in the pertinent art to make and use the invention. The drawings are not to scale.
FIGS. 1A and 1B are perspective and side views, respectively, of exemplary stent-graft prostheses in an expanded or deployed state.
FIG. 2 is a flow chart illustrating steps of a method of forming a stent-graft having graft material formed via two layers of ePTFE according to an embodiment hereof.
FIGS. 2A-2E illustrate step 220 through step 228 of FIG. 2.
FIGS. 3A and 3B are illustrations of the heating elements utilized in FIG. 2.
FIG. 4 is a side view illustration of an alternative method of forming an ePTFE layer.

### DETAILED DESCRIPTION

Disclosed herein is, not describing the part of the invention but for the understanding of the invention, a method of manufacturing a stent-graft prosthesis for implantation within a blood vessel. A first layer of ePTFE is positioned over a mandrel, the mandrel including at least one heating element positioned within a lumen defined by the mandrel. At least one annular stent is positioned onto the first layer of ePTFE, and a second layer of ePTFE is positioned over the stent and the first layer of ePTFE. A heat shrink material is positioned over the second layer of ePTFE such that the heat shrink material fully covers the first and second layers of ePTFE. The first and second layers of ePTFE are heated with the heating element within the mandrel at a temperature to couple together the first and second layers of ePTFE such that the first layer of ePTFE, the at least one stent, and the second layer of ePTFE form the stent-graft prosthesis. The heating step occurs for four minutes or less. The heat shrink material is removed from the stent-graft prosthesis, and then the stent-graft prosthesis is removed from the mandrel.

Also disclosed herein, not describing the part of the invention but for the understanding of the invention, is a method in which a first layer of ePTFE is positioned over a mandrel, and an external heating element is positioned around the mandrel such that an inner surface thereof is spaced apart less than 38.1 mm (1.5 inches) from an outer surface of the mandrel. At least one annular stent is positioned onto the first layer of ePTFE, and a second layer of ePTFE is positioned over the stent and the first layer of ePTFE. A heat shrink material is positioned over the second layer of ePTFE such that the heat shrink material fully covers the first and second layers of ePTFE. The first and second layers of ePTFE are heated with the external heating element at a temperature to couple together the first and second layers of ePTFE such that the first layer of ePTFE, the at least one stent, and the second layer of ePTFE form the stent-graft prosthesis. The heating step occurs for four minutes or less. The heat shrink material is removed from the stent-graft prosthesis, and then the stent-graft prosthesis is removed from the mandrel.

Also disclosed herein, not describing the part of the invention but for the understanding of the invention, is a method in which a first layer of ePTFE is positioned over a mandrel. At least one heating element is positioned within or around the mandrel. At least one annular stent is positioned onto the first layer of ePTFE, and a second layer of ePTFE is positioned over the stent and the first layer of ePTFE. A heat shrink material is positioned over the second layer of ePTFE such that the heat shrink material fully covers the first and second layers of ePTFE. The first and second layers of ePTFE are heated with the heating element at a temperature to entangle the first and second layers of ePTFE on a molecular level such that the first layer of ePTFE, the at least one stent, and the second layer of ePTFE form the stent-graft prosthesis. The stent-graft prosthesis does not include an intermediary melt layer between the first and second layers of ePTFE. The heating step occurs for four minutes or less. The heat shrink material is removed from the stent-graft prosthesis, and then the stent-graft prosthesis is removed from the mandrel.

Specific embodiments of the present invention are now described with reference to the figures, wherein like reference numbers indicate identical or functionally similar elements. The following detailed description is merely exemplary in nature and is not intended to limit the invention or the application and uses of the invention. Although the description of the invention is in the context of a method of making a stent-graft for the treatment of blood vessels, the invention may also be used to make stent-grafts for use in any body passageways where it is deemed useful.

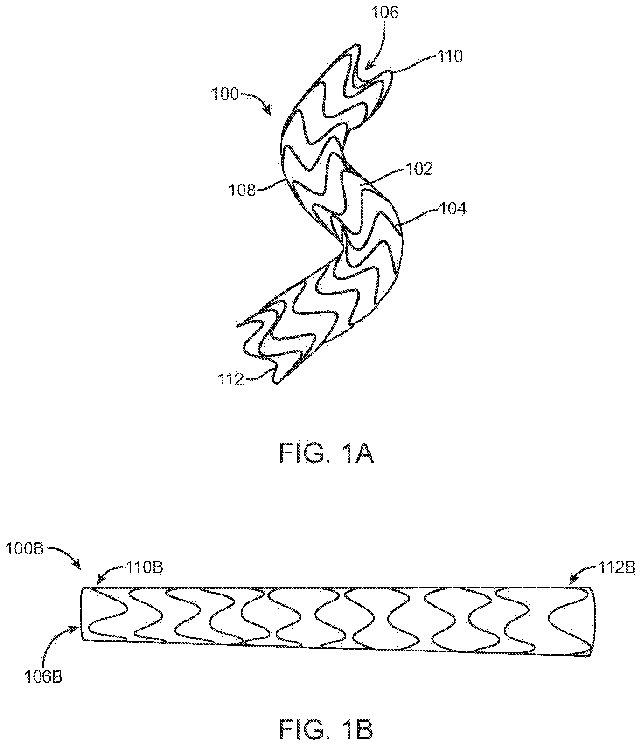

FIG. 1A illustrates a perspective view of a stent-graft prosthesis 100 in a deployed or expanded state or configuration. Stent-graft prosthesis 100 includes a generally tubular or cylindrical body 108 that defines a lumen 106 there through and has a first edge or end 110 and a second edge or end 112. Tubular body 108 includes graft material 102 and at least one radially-compressible stent or scaffold 104 for supporting the graft material and that is operable to self-expand into apposition with an interior wall of a body vessel (not shown). Graft material 102 includes two layers of expanded polytetrafluoroethylene (ePTFE), as will be explained in more detail herein. Stent 104 is constructed from a self-expanding or spring material, and has sufficient radial spring force and flexibility to conformingly engage stent-graft prosthesis 100 with the blood vessel inner wall, to avoid excessive leakage, and prevent pressurization of the aneurysm, i.e., to provide a leak-resistant seal. The term "self-expanding" is used in the following description with reference to one or more stent structures of the prostheses hereof and is intended to convey that the structures are shaped or formed from a material that can be provided with a mechanical memory to return the structure from a compressed or constricted delivery configuration to an expanded deployed configuration. Non-exhaustive exemplary self-expanding materials include stainless steel, a super-elastic metal such as a nickel titanium alloy or Nitinol, various polymers, or a so-called super alloy, which may have a base metal of nickel, cobalt, chromium, or other metal. Mechanical memory may be imparted to a wire or stent structure by thermal treatment to achieve a spring temper in stainless steel, for example, or to set a shape memory in a susceptible metal alloy, such as Nitinol. Various polymers that can be made to have shape memory characteristics may also be suitable for use in embodiments hereof to include polymers such as polynorborene, trans-polyisoprene, styrene-butadiene, and polyurethane. As well poly L-D lactic copolymer, oligo caprylactone copolymer and poly cyclo-octine can be used separately or in conjunction with other shape memory polymers. In another embodiment hereof, stent 104 may be balloon-expandable and formed from a suitable material such as steel.

It will be apparent to one of ordinary skill in the art that the configuration of stent-graft prosthesis 100 is merely exemplary and that the methods of forming a stent-graft prosthesis as described herein may be utilized to form stent-grafts of various other configurations. For example, and not by way of limitation, the support structure or stent of the stent-graft prosthesis may be a unitary tubular stent component having diamond-shaped openings or cells, which may be formed by various conventional stent forming methods as would be understood by one of ordinary skill in the art, rather than a plurality of stents formed as independent sinusoidal patterned rings. In another non-limiting example, the stent may be formed from a wire bent into a waveform and helically wrapped to form the stent, as is known in the art. In addition, the stent-graft prosthesis is not required to have a cylindrical configuration. For example, FIG. 1B illustrates a stent-graft prosthesis 100B having a generally tubular configuration that defines a lumen 106B there through and has a first edge or end 110B and a second flared edge or end 112B, wherein second end 112B has a larger diameter than first end 110B.

Embodiments hereof relate to methods of forming a stent-graft, such as stent-graft prosthesis 100, having graft material formed via two layers of ePTFE with at least one stent being positioned between or encapsulated within the ePTFE layers. The methods disclosed herein utilize one or more direct heating elements that are positioned within or in close proximity to the stent-graft prosthesis, as compared to an air oven. By utilizing one or more heating elements in close proximity to the stent-graft prosthesis, both the temperature and time required to couple the two ePTFE layers together is reduced as compared to baking in an air oven. Reducing the temperature required to couple the two ePTFE layers together is advantageous because minimal temperature induced strain is generated within the stent material. Further, reducing the time required to couple the two ePTFE layers together is advantageous because faster manufacturing times results in cost savings. In addition, the direct heating elements may be utilized to couple the two layers of ePTFE together without an adhesive or melt layer therebetween. ePTFE includes a microstructure of nodes interconnected by fibrils. Quickly heating the ePTFE layers allows the nodes and fibrils of the ePTFE layers to entangle on a molecular level while generating minimal temperature induced strain into the stent material. The elimination of the adhesive or melt layer is advantageous because the melt layer may be difficult and timely to uniformly place over the first ePTFE layer. In addition, when the melt layer is present, the resulting stent-graft prosthesis may be thicker, thus having a greater profile, and stiffer than a stent-graft prosthesis without a melt layer.

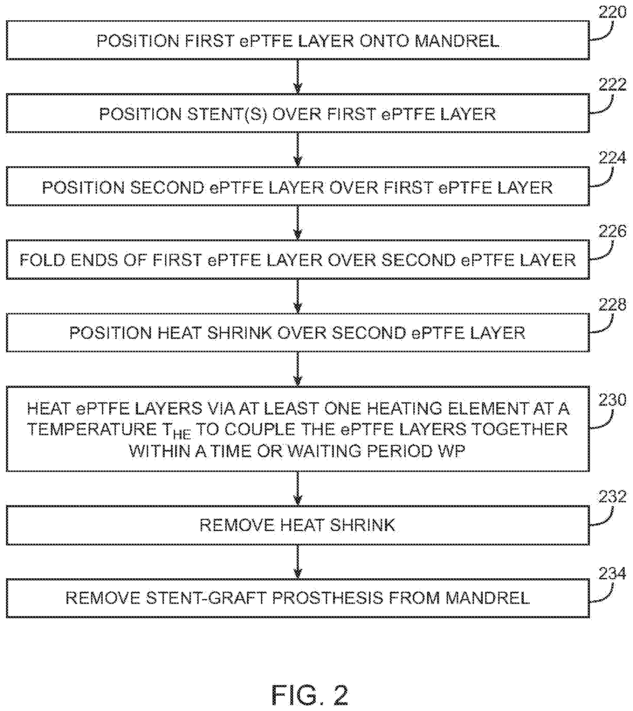

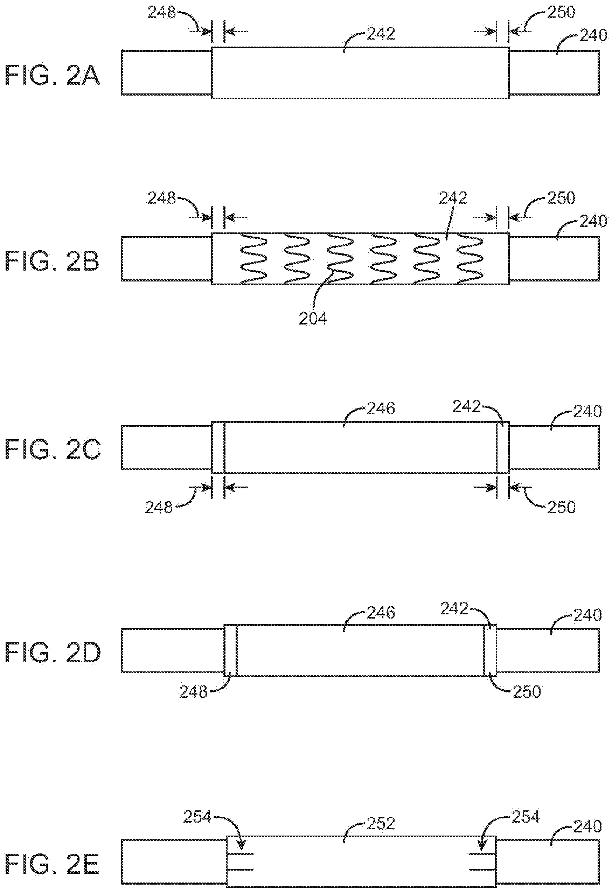

More particularly, FIG. 2 is a flow chart illustrating steps of a method of forming a stent-graft having graft material formed via two layers of ePTFE according to an embodiment hereof. A first layer of ePTFE tubing 242 is positioned over a mandrel 240 as shown in FIG. 2A and step 220 of FIG. 2. The length of ePTFE tubing 242 is greater than the desired length of the stent-graft prosthesis, because ePTFE tubing includes first and second ends 248, 250 which are folded over or cuffed during the manufacturing process as will be explained in more detail herein. Thus, the length of ePTFE tubing is equal to the desired length of the stent-graft prosthesis plus the length of first and second ends 248, 250. In order to place the first layer of ePTFE tubing 242 onto mandrel 240, as well as additional layers of material slid over mandrel 240, mandrel 240 may include a tapered expansion tip (not shown) to ease or assist in positioning of tubing over the mandrel. The first layer of ePTFE tubing 242 is slid over the mandrel until it is approximately centered thereon, as shown in FIG. 2A.

In one embodiment, mandrel 240 is formed of stainless steel and may include a coating on an outer surface thereof. In an embodiment, the coating is electroless nickel-phosphorous plating, commercially available from Bales Mold Service, Inc. of Downers Grove, IL under the tradename Nibore®. Electroless nickel-phosphorous becomes very hard when heated, and thus prevents bonding or adhesion between first layer of ePTFE tubing 242 and mandrel 240 during heating step 230 described herein. In another embodiment, the coating is Kapton®, is a polyimide film developed by DuPont which can remain stable in a wide range of temperatures. In yet another embodiment, available for a method that utilizes only an external heating element as will be described in more detail herein, mandrel 240 may alternatively be formed of polybenzlmidazole. Polybenzlmidazole is an inert biocompatible polymer material with a very high melting point and thus prevents bonding between first layer of ePTFE tubing 242 and mandrel 240 during heating step 230 described herein.

Once first layer of ePTFE tubing 242 in in place, one or more stents 204 are positioned over the first layer of ePTFE tubing as shown in FIG. 2B and step 222 of FIG. 2. Each stent 204 is a sinusoidal patterned ring including a plurality of crowns or bends and a plurality of struts or straight segments with each crown being formed between a pair of opposing struts. Although shown with a series of six independent or separate cylindrical stents, it will be understood by one of ordinary skill in the art that a greater or smaller number of stents may be utilizes depending upon the desired length of the stent-graft prosthesis and/or the intended application thereof. Stents 204 are shown in FIG. 2B has having identical sinusoidal patterns but it will be understood by one of ordinary skill in the art that one or more of stents 204 may have a different pattern or configuration. In an embodiment hereof, stents 204 are positioned onto the first layer of ePTFE at approximately equally-spaced intervals. Stents 204 may be oriented such that the crowns thereof are in line with each other and any joints/seams thereon are in line with each other, although such alignment is not required. Stents 204 are not positioned on first and second ends 248, 250 of first layer of ePTFE tubing 242 because the ends are folded over or cuffed in a later processing step as described in more detail with respect to FIG. 2E. Stents 204 may be self-expanding, as described above with respect to stent 104, or may be balloon-expandable. In another embodiment (not shown), the support structure or stent of the stent-graft prosthesis may be a unitary tubular stent component having diamond-shaped openings or cells rather than a plurality of stents formed as independent sinusoidal patterned rings. In another embodiment (not shown), the stent may be formed from a wire bent into a waveform and helically wrapped to form the stent, as is known in the art

Once stents 204 are in place, a second layer of ePTFE tubing 246 is then positioned directly over the first layer of ePTFE tubing 242 and stents 204 as shown in FIG. 2C and step 224 of FIG. 2, without a melt layer being present between the first and second ePTFE layers such that portions of the ePTFE layers extending between stents 204 abut against each other. The length of ePTFE tubing 246 is approximately equal to the desired length of the stent-graft prosthesis. The second layer of ePTFE tubing 246 is slid over the first layer of ePTFE tubing 242 until it is approximately centered thereon, as shown in FIG. 2D, such that second layer of ePTFE tubing 246 is not positioned on first and second ends 248, 250 of first layer of ePTFE tubing 242. As shown in FIG. 2D and step 226 of FIG. 2, first and second ends 248, 250 are then folded over or cuffed. More particularly, first and second ends 248, 250 are then folded or rolled over such that an outer surface of first and second ends 248, 250 lay against or overlap onto a portion of an outer surface of second layer of ePTFE tubing 246, thereby forming cuffs having three layers of ePTFE on the ends of the assembled components or layers. In one embodiment, first and second ends 248, 250 may each be approximately 5 mm in length.

Once second layer of ePTFE tubing 246 is in place, heat shrink tubing 252 is slid over the second layer of ePTFE tubing 246 until it is approximately centered thereon as shown in FIG. 2E and step 228 of FIG. 2. As will be understood by one of ordinary skill in the art, heat shrink tubing is expanded tubing that will shrink when heat is applied thereto in an effort to return or recover to the relaxed diameter it originally had when extruded. When heat shrink tubing 252 shrinks or recovers, it radially compresses the assembled components or layers during heating step 230 described herein to form the stent-graft prosthesis in a compressed or delivery configuration suitable for delivery into the vasculature. The length of heat shrink tubing 252 is greater than the length of second layer of ePTFE tubing 246 such that the heat shrink material completely/fully covers or extends over the assembled components or layers on mandrel 240. Heat shrink tubing 252 may include one or more slits or cuts 254, formed or made transverse to a longitudinal axis of the heat shrink tubing, at a first and/or second end or edge thereof to facilitate the step of removing the heat shrink material as will be described in more detail herein. Heat shrink tubing 252 is formed of a polymeric material such as polytetrafluoroethylene (PTFE) and may include a coating coupled to or on an inner surface thereof. In one embodiment, the coating is high temperature resistant parylene HT (high temperature), commercially available from Specialty Coatings Systems of Indianapolis, Indiana, which prevents bonding/adhesion between second layer of ePTFE tubing 246 and heat shrink tubing 252 during heating step 230 described herein. The parylene HT coating has a thickness not greater than five microns or micrometers in order to conform to the inner surface of heat shrink tubing 252, and in one embodiment, the parylene HT coating has a thickness not greater than one micron or micrometer. In addition to preventing bonding/coupling between second layer of ePTFE tubing 246 and heat shrink tubing 252 during the heating cycle, the parylene HT coating also results in a stent-graft prosthesis having a smooth, substantially wrinkle-free outer surface. In another embodiment hereof, rather than a coating on the inner surface of the heat shrink tubing, a layer of aluminum foil may be utilized as a lining that extends along an inner surface of heat shrink tubing 252 to prevent bonding/coupling between second layer of ePTFE tubing 246 and heat shrink tubing 252 during heating step 230 described herein.

With first and second ePTFE layers 242, 246, stents 204, and heat shrink material 252 assembled onto mandrel 240, at least one direct heating element or source at a required or prescribed temperature T_{HE} is utilized to heat first and second ePTFE layers 242, 246 as shown in step 230 of FIG. 2. With the heating element at temperature T_{HE}, the ePTFE layers 242, 246 are heated such that the nodes and fibrils of the ePTFE material entangle, intertwine, interweave, or otherwise mesh together, thereby coupling the first and second ePTFE layers together without an intermediary layer therebetween. The heating step continues for a required time or waiting period WP such that first and second ePTFE layers 242, 246 entangle, thereby forming the stent-graft prosthesis. As used herein, required time or waiting period WP begins or occurs after the heating element has warmed up to temperature T_{HE} and the equipment/heating element is considered to be at a steady state temperature of the prescribed/required temperature T_{HE}.

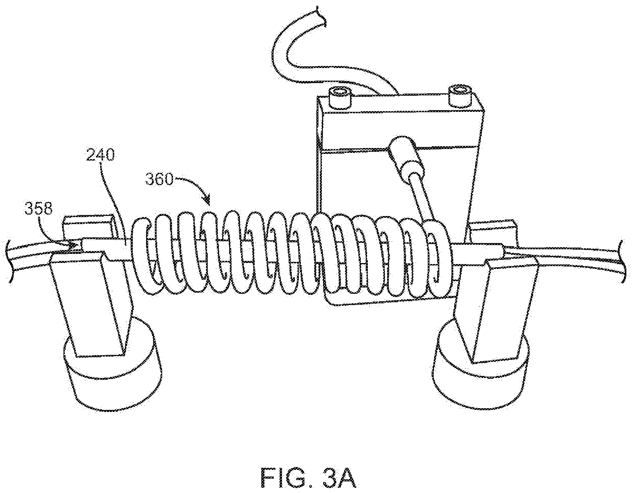

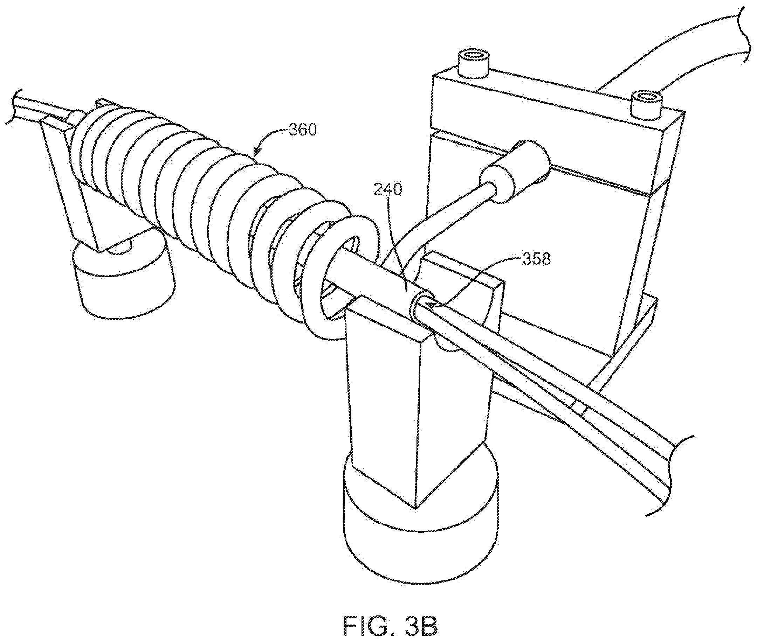

Accordingly to embodiments hereof, temperature T_{HE} and waiting period WP depend upon the type of and proximity of the heating element(s) utilized herein. As illustrated in FIGS. 3A and 3B, in one embodiment hereof, two heating elements are utilized in heating step 230. The two heating elements include a first internal heating element 358 positioned within a lumen (not shown) of mandrel 240 and a second external heating element 360 surrounding mandrel 240 and positioned in close proximity thereto. In an embodiment hereof, the temperature T_{HE} of internal heating element 358 is 250-290 °C and the temperature T_{HE} of external heating element 360 is approximately 360 °C. As used herein, approximately 360 °C includes temperatures ranging between 340-380 °C. Waiting period WP, *i.e*., the period of time it takes first and second ePTFE layers 242, 246 to couple together or entangle, is two minutes or less. In one embodiment, waiting period WP is approximately one minute.

The relatively lower temperatures and relatively faster heating times described herein are due to the proximity of heating elements 358, 360. The proximity of heating elements 358, 360 allows the assembled components or layers on mandrel 240 to be heated using conduction, rather than convection as utilized in air ovens. By placing heating elements into and/or close to mandrel 240, heat can travel directly to first and second ePTFE layers 242, 246 to result in the short waiting periods WP described herein. In addition, the proximity of external heating element 360 results in a quick recovery or shrinking of heat shrink tubing 254 during the manufacturing process. Notably, the structural configurations of heating elements 358, 360 described herein are not required but rather the placement and described proximity of the heating element(s) is necessary to heat the ePTFE layers via conduction within the described temperatures T_{HE} and waiting periods WP.

Internal heating element 358, which is commercially available from manufacturers such as but not limited to Watlow Electric Manufacturing Company, is positioned within a lumen (not shown) of mandrel 240 and heats mandrel 240. When mandrel 240 is formed from conductive stainless steel, mandrel 240 reaches approximately the same temperature as heating element 358. External heating element 360, which is commercially available from manufacturers such as but not limited to Watlow Electric Manufacturing Company, circumferentially surrounds mandrel 240 and is positioned in close proximity thereto. For example, in the depicted embodiment, external heating element 360 is a continuous coil or helix having an inner surface that is spaced apart between 0.508 to 38.1 mm (0.02 to 1.5 inches) from an outer surface of mandrel 240. The coil configuration of external heating element 360 is not required but it is necessary for the external heating element to be in close proximity to an outer surface of mandrel 240 as described herein. For example, rather than a coil configuration for external heating element 360, a heated metal die or tool having an inner surface that is heated to the temperatures T_{HE} and is spaced apart between 0.508 to 38.1 mm (0.02 to 1.5 inches) from an outer surface of mandrel 240 may be utilized as external heating element 360. Heated metal dies or tools are known in the art of hot forming and may utilize a plurality of electrical heaters in separate electrically powered and controlled heating zones which may be very effective in providing close control of the temperature of the heated inner surface.

Although the fastest bonding times are achieved by utilizing both internal and external heating elements as described above, either heating element may be solely utilized in heating step 230. For example, in one embodiment hereof, only internal heating element 358 in utilized in heating step 230. In this embodiment hereof, the temperature T_{HE} of internal heating element 358 is between 250-290 °C and waiting period WP, *i.e.,* the period of time it takes first and second ePTFE layers 242, 246 to couple together, is four minutes or less. When internal heating element 358 is the only heating element or source used, mandrel 240 may be formed of stainless steel and may include an electroless nickel-phosphorous or Kapton® coating as described above to prevent bonding between first layer of ePTFE tubing 242 and mandrel 240 during the heating step. In another embodiment hereof, only external heating element 360 is utilized in heating step 230. In this embodiment hereof, the temperature T_{HE} of external heating element 360 is approximately 360 °C and waiting period WP, i.e., the period of time it takes first and second ePTFE layers 242, 246 to couple together, is four minutes or less. When external heating element 360 is the only heating element or source used, mandrel 240 may be formed of polybenzlmidazole or may be formed of stainless steel with a coating thereon to prevent bonding between first layer of ePTFE tubing 242 and mandrel 240 during heating step 230 described herein. Polybenzlmidazole does not allow for heat transfer and thus would not be utilized as a material for mandrel 240 in any embodiment described herein in which an internal heating element is used within the mandrel.

Once first and second ePTFE layers 242, 246 are entangled or coupled together, a stent-graft prosthesis is thereby formed. The stent-graft prosthesis has been radially compressed due to shrinkage of heat shrink tubing 252 and includes the first ePTFE layer 242, stents 204, and the ePTFE second layer 246. Heat shrink tubing 252 may then be removed as shown in step 232 of FIG. 2. In one embodiment, heat shrink tubing 252 may be ripped or torn off the stent-graft prosthesis by pulling at slits 254 formed therein. When heat shrink tubing 252 includes a parylene HT coating on the inner surface thereof as described above, heat shrink tubing 252 advantageously cracks or snaps apart during removal thereof. After heat shrink tubing 252 is removed, the stent-graft prosthesis may also be removed from mandrel 240 as shown in step 234 of FIG. 2. The stent-graft prosthesis may be removed from the mandrel by pulling or sliding it off.

Although the embodiment described above with respect to FIG. 2 describes that first and second ePTFE layers 242, 246 are directly or immediately entangled together without an adhesive or melt layer therebetween, it will be understood by those of ordinary skill in the art that the direct heating elements described herein may be utilized to form a stent-graft prosthesis having an intermediary adhesive or melt layer applied or positioned between first and second ePTFE layers 242, 246. The melt layer may be a thermoplastic material that operates or functions to bond or tie the two layers of ePTFE together such as but not limited to fluoroethylpolypropylene (FEP), polytetrafluoroethylene, polyurethane, polyamide, polyimide or silicone. If an intermediary melt layer is present, the required time or waiting period required to bond first and second ePTFE layers 242, 246 together via melting or reflowing of the adhesive layer is equal to or less than the waiting periods WP described herein.

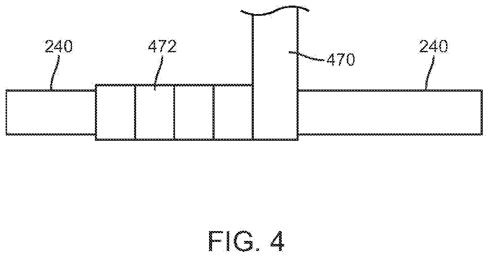

In addition, although the embodiment described above with respect to FIG. 2 describes each of first and second ePTFE layers 242, 246 as tubular components, it will be understood by those of ordinary skill in the art that first and/or second ePTFE layers 242, 246 may be formed via ePTFE material which is not originally formed as a tubular component. For example, as shown in FIG. 4, first and/or second ePTFE layers 242, 246 may be formed via an elongated ribbon or band 470 of ePTFE that is wrapped around the outer surface of mandrel 240. Band 470 is wrapped or wound around mandrel 240 in a helical or corkscrew fashion to form a series of loops 472. Adjacent edges of loops 472 may slightly overlap or may abut against each other. In one embodiment, when forming second ePTFE layer 246, adjacent edges of loops 472 may be slightly spaced apart so long as loops 472 completely cover or extend over stent(s) 204. Gaps between loops 472 may provide the stent-graft prosthesis with additional flexibility while stent(s) 204 are still encapsulated by the ePFTE layers.

Further, although the embodiment described above with respect to FIG. 2 describes each of first and second ePTFE layers 242, 246 as single tubular components, it will be understood by those of ordinary skill in the art that first layer 242 may include one or more tubular ePTFE layers or components and/or second layer 246 may include one or more tubular ePTFE layers or components. If more than two layers of ePTFE are included, heating step 230 as described herein operates to entangle all layers of ePTFE together within the above-described waiting periods such that all of the ePTFE layers and the stent(s) form a stent-graft prosthesis.

While various embodiments according to the present invention have been described above, it should be understood that they have been presented by way of illustration and example only, and not limitation. The breadth and scope of the present invention should not be limited by any of the above-described exemplary embodiments, but should be defined only in accordance with the appended claims.

## Claims

1. A method of manufacturing a stent-graft prosthesis for implantation within a blood vessel, the method comprising the steps of:
positioning a first layer of ePTFE over a mandrel, wherein the mandrel includes an internal heating element (358) positioned within a lumen defined by the mandrel, the internal heating element being the only heating element utilized;
positioning at least one annular stent onto the first layer of ePTFE;
positioning a second layer of ePTFE over the stent and the first layer of ePTFE;
positioning a heat shrink material over the second layer of ePTFE, wherein the heat shrink material fully covers the first and second layers of ePTFE;
heating the first and second layers of ePTFE with the heating element within the mandrel at a temperature between 250-290 °C to couple together the first and second layers of ePTFE such that the first layer of ePTFE, the at least one stent, and the second layer of ePTFE form the stent-graft prosthesis, wherein the heating step occurs for four minutes or less;
removing the heat shrink material; and
removing the stent-graft prosthesis from the mandrel.

2. The method of claim 1, wherein the stent-graft prosthesis does not include an intermediary melt layer between the first and second layers of ePTFE and the heating step includes entangling the first and second layers of ePTFE on a molecular level.

3. The method of claim 1, wherein the mandrel is formed from stainless steel and includes an electroless nickel-phosphorous coating on an outer surface thereof.

4. The method of claim 1, wherein the heat shrink material is PTFE and includes a parylene coating on an inner surface thereof.

5. The method of claim 1, wherein the step of positioning at least one annular stent onto the first layer of ePTFE includes positioning a plurality of annular stents onto the first layer of ePTFE at approximately equally-spaced intervals.

6. The method of claim 1, further comprising at least one of:
the step of folding end portions of the first layer of ePFTE over an outer surface of the second layer of ePTFE prior to the step of positioning the heat shrink material over the second layer of ePTFE and the step of forming small slits in at least one edge of the heat shrink material to facilitate the step of removing the heat shrink material.

7. A method of manufacturing a stent-graft prosthesis for implantation within a blood vessel, the method comprising the steps of:
positioning a first layer of ePTFE over a mandrel, wherein the mandrel includes at least one heating element positioned within a lumen defined by the mandrel;
positioning at least one annular stent onto the first layer of ePTFE;
positioning a second layer of ePTFE over the stent and the first layer of ePTFE;
positioning a heat shrink material over the second layer of ePTFE, wherein the heat shrink material fully covers the first and second layers of ePTFE;
heating the first and second layers of ePTFE with the heating element within the mandrel at a temperature between 250-290 °C to couple together the first and second layers of ePTFE such that the first layer of ePTFE, the at least one stent, and the second layer of ePTFE form the stent-graft prosthesis, and
heating the first and second layers of ePTFE with an external heating element at a second temperature of 340-380 °C, the external heating element positioned around the mandrel, the external heating element having an inner surface that is spaced apart less than 38.1 mm (1.5 inches) from an outer surface of the mandrel;
removing the heat shrink material; and
removing the stent-graft prosthesis from the mandrel; wherein
the heating step occurs for two minutes or less.

8. The method of claim 7, wherein the mandrel is formed from polybenzlmidazole.

9. The method of claim 7, wherein the stent-graft prosthesis does not include an intermediary melt layer between the first and second layers of ePTFE and the heating step includes entangling the first and second layers of ePTFE on a molecular level.

10. The method of claim 7, wherein the heat shrink material is PTFE and includes a parylene coating on an inner surface thereof.

11. The method of claim 9, wherein the second temperature is approximately 360 °C.

12. The method of claim 7, wherein the external heating element circumferentially surrounds the mandrel.

## Patentansprüche

1. Verfahren zur Herstellung einer Stentgraft-Prothese zur Implantation innerhalb eines Blutgefäßes, wobei das Verfahren die Schritte umfasst:
Positionieren einer ersten Schicht aus ePTFE über einem Mandrin, wobei der Mandrin ein innenliegendes Heizelement (358) aufweist, das innerhalb eines Lumens, das durch den Mandrin definiert ist, positioniert ist, wobei das innenliegende Heizelement das einzige verwendete Heizelement ist;
Positionieren mindestens eines ringförmigen Stents auf der ersten Schicht aus ePTFE;
Positionieren einer zweiten Schicht aus ePTFE über dem Stent und der ersten Schicht aus ePTFE;
Positionieren eines Wärmeschrumpfmaterials über der zweiten Schicht aus ePTFE, wobei das Wärmeschrumpfmaterial die erste und die zweite Schicht aus ePTFE vollständig bedeckt;
Erhitzen der ersten und der zweiten Schicht aus ePTFE mit dem Heizelement innerhalb des Mandrins bei einer Temperatur zwischen 250-290 °C, um die erste und die zweite Schicht aus ePTFE derart miteinander zu koppeln, dass die erste Schicht aus ePTFE, der mindestens eine Stent und die zweite Schicht aus ePTFE die Stentgraft-Prothese bilden, wobei der Erhitzungsschritt für vier Minuten oder weniger erfolgt;
Entfernen des Wärmeschrumpfmaterials; und
Entfernen der Stentgraft-Prothese von dem Mandrin.

2. Verfahren nach Anspruch 1, wobei die Stentgraft-Prothese keine Zwischenschmelzschicht zwischen der ersten und der zweiten Schicht aus ePTFE einschließt und der Erhitzungsschritt ein Verwickeln der ersten und zweiten Schicht aus ePTFE auf einer molekularen Ebene einschließt.

3. Verfahren nach Anspruch 1, wobei der Mandrin aus rostfreiem Stahl gebildet ist und eine stromlose Nickelphosphor-Beschichtung auf seiner Außenoberfläche einschließt.

4. Verfahren nach Anspruch 1, wobei das Wärmeschrumpfmaterial PTFE ist und eine Parylen-Beschichtung auf seiner Innenoberfläche einschließt.

5. Verfahren nach Anspruch 1, wobei der Schritt des Positionierens mindestens eines ringförmigen Stents auf der ersten Schicht aus ePTFE das Positionieren einer Vielzahl von ringförmigen Stents auf der ersten Schicht aus ePTFE bei etwa gleichmäßig beabstandeten Intervallen einschließt.

6. Verfahren nach Anspruch 1, ferner umfassend mindestens eines von:
dem Schritt des Faltens von Endabschnitten der ersten Schicht aus ePFTE über eine Außenoberfläche der zweiten Schicht aus ePTFE vor dem Schritt des Positionierens des Wärmeschrumpfmaterials über der zweiten Schicht aus ePTFE und dem Schritt des Bildens kleiner Schlitze in mindestens einem Rand des Wärmeschrumpfmaterials, um den Schritt des Entfernens des Wärmeschrumpfmaterials zu erleichtern.

7. Verfahren zur Herstellung einer Stentgraft-Prothese zur Implantation innerhalb eines Blutgefäßes, wobei das Verfahren die Schritte umfasst:
Positionieren einer ersten Schicht aus ePTFE über einem Mandrin, wobei der Mandrin mindestens ein Heizelement einschließt, das innerhalb eines Lumens, das durch den Mandrin definiert ist, positioniert ist;
Positionieren mindestens eines ringförmigen Stents auf der ersten Schicht aus ePTFE;
Positionieren einer zweiten Schicht aus ePTFE über dem Stent und der ersten Schicht aus ePTFE;
Positionieren eines Wärmeschrumpfmaterials über der zweiten Schicht aus ePTFE, wobei das Wärmeschrumpfmaterial die erste und die zweite Schicht aus ePTFE vollständig bedeckt;
Erhitzen der ersten und der zweiten Schicht aus ePTFE mit dem Heizelement innerhalb des Mandrins bei einer Temperatur zwischen 250-290 °C, um die erste und die zweite Schicht aus ePTFE derart miteinander zu koppeln, dass die erste Schicht aus ePTFE, der mindestens eine Stent und die zweite Schicht aus ePTFE die Stentgraft-Prothese bilden, und
Erhitzen der ersten und der zweiten Schicht aus ePTFE mit einem außenliegenden Heizelement bei einer zweiten Temperatur von 340-380 °C, wobei das außenliegende Heizelement um den Mandrin herum positioniert ist, wobei das außenliegende Heizelement eine Innenoberfläche aufweist, die von einer Außenoberfläche des Mandrins weniger als 38,1 mm (1,5 Zoll) beabstandet ist;
Entfernen des Wärmeschrumpfmaterials; und
Entfernen der Stentgraft-Prothese von dem Mandrin; wobei
der Erhitzungsschritt für zwei Minuten oder weniger erfolgt.

8. Verfahren nach Anspruch 7, wobei der Mandrin aus Polybenzimidazol gebildet ist.

9. Verfahren nach Anspruch 7, wobei die Stentgraft-Prothese keine Zwischenschmelzschicht zwischen der ersten und der zweiten Schicht aus ePTFE einschließt und der Erhitzungsschritt ein Verwickeln der ersten und zweiten Schicht aus ePTFE auf einer molekularen Ebene einschließt.

10. Verfahren nach Anspruch 7, wobei das Wärmeschrumpfmaterial PTFE ist und eine Parylen-Beschichtung auf seiner Innenoberfläche einschließt.

11. Verfahren nach Anspruch 9, wobei die zweite Temperatur etwa 360 °C beträgt.

12. Verfahren nach Anspruch 7, wobei das außenliegende Heizelement den Mandrin in Umfangsrichtung umgibt.

## Revendications

1. Procédé de fabrication d'une prothèse de type endoprothèse-greffon pour implantation à l'intérieur d'un vaisseau sanguin, le procédé comprenant les étapes consistant à :
positionner une première couche d'ePTFE sur un mandrin, dans lequel le mandrin inclut un élément chauffant interne (358) positionné à l'intérieur d'une lumière définie par le mandrin, l'élément chauffant interne étant le seul élément chauffant utilisé ;
positionner au moins une endoprothèse annulaire sur la première couche d'ePTFE ;
positionner une deuxième couche d'ePTFE sur l'endoprothèse et la première couche d'ePTFE ;
positionner un matériau thermorétractable sur la deuxième couche d'ePTFE, dans lequel le matériau thermorétractable recouvre entièrement les première et deuxième couches d'ePTFE ;
chauffer les première et deuxième couches d'ePTFE avec l'élément chauffant à l'intérieur du mandrin à une température comprise entre 250 et 290 °C afin de coupler l'une à l'autre les première et deuxième couches d'ePTFE de telle sorte que la première couche d'ePTFE, l'au moins une endoprothèse et la deuxième couche d'ePTFE forment la prothèse de type endoprothèse-greffon, dans lequel l'étape de chauffage a lieu pendant quatre minutes ou moins ;
retirer le matériau thermorétractable ; et
retirer la prothèse de type endoprothèse-greffon du mandrin.

2. Procédé selon la revendication 1, dans lequel la prothèse de type endoprothèse-greffon n'inclut pas une couche de fusion intermédiaire entre les première et deuxième couches d'ePTFE et l'étape de chauffage inclut l'enchevêtrement des première et deuxième couches d'ePTFE à un niveau moléculaire.

3. Procédé selon la revendication 1, dans lequel le mandrin est formé d'acier inoxydable et inclut un revêtement de nickel-phosphore autocatalytique sur une surface externe de celui-ci.

4. Procédé selon la revendication 1, dans lequel le matériau thermorétractable est du PTFE et inclut un revêtement de parylène sur une surface interne de celui-ci.

5. Procédé selon la revendication 1, dans lequel l'étape consistant à positionner au moins une endoprothèse annulaire sur la première couche d'ePTFE inclut le positionnement d'une pluralité d'endoprothèses annulaires sur la première couche d'ePTFE à des intervalles espacés approximativement de manière égale.

6. Procédé selon la revendication 1, comprenant en outre au moins une étape parmi :
l'étape consistant à plier des parties d'extrémité de la première couche d'ePTFE sur une surface externe de la deuxième couche d'ePTFE avant l'étape consistant à positionner le matériau thermorétractable sur la deuxième couche d'ePTFE et l'étape consistant à former des petites fentes dans au moins un bord du matériau thermorétractable afin de faciliter l'étape consistant à retirer le matériau thermorétractable.

7. Procédé de fabrication d'une prothèse de type endoprothèse-greffon pour implantation à l'intérieur d'un vaisseau sanguin, le procédé comprenant les étapes consistant à :
positionner une première couche d'ePTFE sur un mandrin, dans lequel le mandrin inclut au moins un élément chauffant positionné à l'intérieur d'une lumière définie par le mandrin ;
positionner au moins une endoprothèse annulaire sur la première couche d'ePTFE ;
positionner une deuxième couche d'ePTFE sur l'endoprothèse et la première couche d'ePTFE ;
positionner un matériau thermorétractable sur la deuxième couche d'ePTFE, dans lequel le matériau thermorétractable recouvre entièrement les première et deuxième couches d'ePTFE ;
chauffer les première et deuxième couches d'ePTFE avec l'élément chauffant à l'intérieur du mandrin à une température comprise entre 250 et 290 °C afin de coupler l'une à l'autre les première et deuxième couches d'ePTFE de telle sorte que la première couche d'ePTFE, l'au moins une endoprothèse et la deuxième couche d'ePTFE forment la prothèse de type endoprothèse-greffon, et
chauffer les première et deuxième couches d'ePTFE avec un élément chauffant externe à une deuxième température allant de 340 à 380 °C, l'élément chauffant externe étant positionné autour du mandrin, l'élément chauffant externe ayant une surface interne qui est espacée de moins de 38,1 mm (1,5 pouce) d'une surface externe du mandrin ;
retirer le matériau thermorétractable ; et
retirer la prothèse de type endoprothèse-greffon du mandrin ; dans lequel
l'étape de chauffage a lieu pendant deux minutes ou moins.

8. Procédé selon la revendication 7, dans lequel le mandrin est formé de polybenzimidazole.

9. Procédé selon la revendication 7, dans lequel la prothèse de type endoprothèse-greffon n'inclut pas une couche de fusion intermédiaire entre les première et deuxième couches d'ePTFE et l'étape de chauffage inclut l'enchevêtrement des première et deuxième couches d'ePTFE à un niveau moléculaire.

10. Procédé selon la revendication 7, dans lequel le matériau thermorétractable est du PTFE et inclut un revêtement de parylène sur une surface interne de celui-ci.

11. Procédé selon la revendication 9, dans lequel la deuxième température est d'approximativement 360 °C.

12. Procédé selon la revendication 7, dans lequel l'élément chauffant externe entoure circonférentiellement le mandrin.
